# EUROPEAN PATENT APPLICATION

(11) **EP 1 712 609 A1**
(43) Date of publication of application: **18.10.2006**
(21) Application number: 06007512.4
(22) Date of filing: 10.04.2006
(51) Int. Cl.: C11C 1/00, C11C 3/14, C07C 51/487, C07C 51/43, C07C 57/12

(54) **Method for preparing unsaturated fatty acids**

(30) Priority: 11.04.2005 KR 20050029829
(71) Applicant: Dongbu Hannong Chemical Co., Ltd., Seoul 135-523 (KR)
(72) Inventor: Sung, Soon-Kee, Yuseong-gu Daejeon-si 305-708 (KR); Lee, Seong Kweon, Yuseong-gu Daejeon-si 305-708 (KR)
(74) Representative: Grünecker, Kinkeldey, Stockmair & Schwanhäusser Anwaltssozietät

(57) **Abstract**

Disclosed relates to a method for preparing unsaturated fatty acids and, more particularly, to a method for preparing unsaturated fatty acids in a high purity of at least 99% by isolating and purifying unsaturated fatty acids via a secondary nucleation mechanism using fatty acid-urea inclusion compounds.

## Description

### BACKGOUND OF THE INVENTION

### 1. Field of the Invention

The present invention relates to a method for preparing unsaturated fatty acids and, more particularly, to a method for preparing unsaturated fatty acids in a high purity of at least 99% by isolating and purifying fatty acids via a secondary nucleation mechanism using fatty acid-urea inclusion compounds.

### 2. Description of Related Art

Various animal and vegetable fatty oils, for example, vegetable oils such as safflower oil, sunflower oil, olive oil, etc. and fish oils such as sardine oil, contain saturated and unsaturated fatty acids having valuable effects for the food and medicinal purpose. Especially, the unsaturated fatty acid among the fat and oil fatty acids has attracted attention since it has various valuable effects for the food and medicinal purpose.

Typically, methods for converting fatty oils into fatty acids are classified roughly into an enzyme therapy and an organic synthetic method. The conversion ratio of the enzyme therapy is no more than 70% to 80% and it is difficult to expect the conversion into valid fatty acids having valuable effects for the food and medicinal purpose through the enzyme therapy. Conjugated linoleic acid is a typical example. Besides, the conversion ratio of the organic synthetic method is about 98%, however, it is attended with difficulties in isolating and purifying the fatty acids due to carcinogenic hazard of trans-9,trans-11 fatty acids produced during the conversion. The trans-9,trans-11 fatty acids, which are carcinogenic materials, are unavoidable by-products because the organic synthetic method puts emphasis on isomerizing the fatty acids at high temperature, caring nothing for the cooling of the aftertreatment process. In addition, it is unavoidable that unreacted fatty acids remain in this step. Accordingly, it is necessary to isolate and purify unsaturated fatty acids in a high purity from the animal and vegetable fatty oils in order to utilize such unsaturated fatty acids as raw materials for food and pharmaceutical products. Various researches aimed at improving the method for removing such harmful materials continue to progress.

Urea inclusion compound methods have been widely known as a method for isolating and purifying unsaturated fatty acids from the animal and vegetable fat and oil fatty acids. One of these methods, an alcoholic-liquid cooling method for simultaneously dissolving fatty acids and urea has been known in numerous references (JAOCS, 59, 117.about.118 (March 1982), Haagsma; Ratnayake, Fatsci. Technol. 90, 381 (1998), etc.). However, since such cooling method cannot control the size of urea molecular group, urea and urea inclusion compound are simultaneously deposited in the form of crystal during the cooling step, thus sharply deteriorating the efficiency of urea, which results in drawbacks that unnecessary fatty acids are not removed. Accordingly, the conventional alcoholic-liquid cooling method has been applied to isolate unsaturated fatty acids in a mid purity rather than in a high purity. In addition, there has been raised the necessity to lower the cooling rate in order to overcome such drawbacks of the conventional method.

However, according to the method in which the cooling rate is lowered, it has also some drawbacks that the process is made slow, and the rancidity proceeds rapidly due to the long stay of unsaturated fatty acids at high temperature, thus decreasing the oxidative stability of fatty acids. Accordingly, such method deteriorating the quality of the products cannot be applied in a mass production.

To overcome such drawbacks, Korean Patent Laid-Open No. 10-2002-0042432 has disclosed a method for isolating and purifying unsaturated fatty acids in a high purity by performing a urea inclusion crystallization step and, then, selectively utilizing a cooling crystallization or a high performance liquid chromatography. It is possible to apply such method economically, however, it is impossible to give a perfectly high resolution since it controls merely the behavior of the urea molecular group by the cooling step of the method. In addition, it cannot avoid the precipitation of urea crystals and the time required for processing becomes longer since it applies several steps such as the cooling crystallization, the high liquid chromatography, besides the urea inclusion crystallization, which are additional drawbacks of the conventional method.

Thus, the present inventors have studied methods for preparing unsaturated fatty acids in a high purity from fat and oil fatty acids and complete the present invention that enhances the oxidative stability of fatty acids by shortening the staying time of unsaturated fatty acids at high temperature using only the urea inclusion crystallization step and, selectively isolates and purifies desired fatty acids without precipitation of urea crystals by controlling the behavior of the urea molecular group via a secondary nucleation mechanism using fatty acid-urea inclusion compounds, thus obtaining unsaturated fatty acids in a high purity of at least of 99%.

### SUMMARY OF THE INVENTION

Accordingly, an object of the present invention is to provide a method for preparing unsaturated fatty acids in a high purity of at least 99% and by isolating and purifying fatty acids via a secondary nucleation mechanism using fatty acid-urea inclusion compounds.

The method for preparing unsaturated fatty acids of the invention comprises the steps of: (1) putting NaOH into C₁~C₅ alcohol to be completely dissolved, adding fat and oil fatty acids to the resulting solution and cooling the resulting reactant to be converted into fatty acids; (2) adding urea to ethanol solution to be completely dissolved and adding the fatty acids obtained in step (1) to the resulting solution to form fatty acid-urea inclusion compounds; and (3) mixing ethanol solution, urea and fatty acids to be completely dissolved, cooling the resulting solution and, at the same time, adding the fatty acid-urea inclusion compounds obtained in step (2) to the resulting reactant to be reacted to obtain fatty acids.

The method for preparing unsaturated fatty acids of the invention will be described in steps hereinafter.

In step (1), 0.25 to 0.5 weight fractions of NaOH are added to 1.0 weight fraction of C₁~C₅ alcohol to be completely dissolved. Keeping the temperature of the reactant within 170°C to 180°C, 1.0 to 2.0 weight fractions of fat and oil fatty acids are added to the solution to be isomerized at such high temperature for one and a half to two (1.5 to 2) hours. Then, the isomerization step is terminated by cooling the resulting reactant at temperature of 50°C to 60°C. With such cooling step, it is possible to convert unreacted fatty acids into unsaturated fatty acids of more than 99% and to minimize the formation of trans-9,trans-11 fatty acids, which are carcinogenic hazardous substances. In case that a high-speed cooling method is carried out, it is possible to suppress the formation of trans-9,trans-11 fatty acids, however, it cannot proceed further with the conversion of unreacted fatty acids into unsaturated fatty acids. To the contrary, in case that a low-speed cooling method or a controlled cooling method is provided, it is possible to cause complete conversion of unreacted fatty acids into unsaturated fatty acids completely, however, it cannot avoid the formation of trans-9,trans-11 fatty acids due to continuous heat generated. Accordingly, it can be understood that such cooling conditions are the most important factor to overcome the above-mentioned problems.

The above described C₁~C₅ alcohol includes methylene glycol, ethylene glycol, propylene glycol, butylene glycol and pentylene glycol. Preferably, propylene glycol may be used.

When the temperature of the reactant reaches 50°C to 60°C, phosphoric acids are added slowly to the reactant to adjust the pH value to 1. After stationing under the present condition, the lower part of the reactant is removed and the fatty acid layer in the upper part is washed by distilled water. This process is carried out two to three times to remove impurities dissolved in water completely. The fatty acids obtained in this step contain unreacted fatty acids of 0.2% to 0.5% and trans-9,trans-11 fatty acids of 1.0% or less.

The fat and oil fatty acids contains various animal and vegetable fatty oils, for example, vegetable oils such as safflower oil, sunflower oil, olive oil, etc. and fish oils such as sardine oil and the like.

The fatty acids obtained in step (1) are preferably unsaturated fatty acids having 10 to 30 carbon atoms and 1 to 6 bonds and, more preferably, comprise at least one selected from the group consisting of a myristoleic acid, a palmitoleic acid, a γ-linolenic acid, an α-linolenic acid, an oleic acid, a linoleic acid, a conjugated linoleic acid, a docosahexaenoic acid(DHA) and an eicosapentaenoic acid (EPA) .

In step (2), 1.0 weight fraction of urea is mixed with 3.0 to 5.0 weight fractions of ethanol solution to be completely dissolved at temperature of 25°C to 30°C. Keeping this temperature, 0.2 to 0.4 weight fractions of fatty acids obtained in step (1) is added to the solution in order to form fatty acid-urea inclusion compounds. Subsequently, the resulting crystals are filtered and dried for the use as nucleuses of the following secondary nucleation mechanism. Here, the ethanol solution may be used in concentration of 60% to 80%, preferably, in concentration of 70%.

In step (3), 9.0 to 12.0 weight fractions of ethanol solution, 3.0 to 4.0 weigh parts of urea and 1.0 weight fraction of fatty acid are mixed to be completely dissolved at temperature of 60°C to 70°C. Then, the solution is cooled at cooling rate of 0.5 to 1.0°C/min, to which 0.01 to 0.05 weight fractions of fatty acid-urea inclusion compounds obtained in step (2) are added at temperature of 45°C to 50°C. Here, it is most desired to keep the form of crystals, not being dissolved. Under the condition that the crystals are present, the reactant are cooled at temperature of 10°C to 15°C and filtered. After identifying the portion of the crystals and the solution by a gas chromatography analyzer, the portion where fatty acid-urea inclusion compounds are shown is taken. Then, water and hexane in the weight ratio of 1:1 are added to the crystals (or the portion of the solution) and, subsequently, a small amount of hydrochloric acid is added to them to be mixed, thus forming fatty acids containing hexane in the upper part thereof. Consequently, concentrated fatty acids in the range of 60% to 99% can be obtained by removing the hexane in the upper part. Here, the ethanol solution may be used in concentration of 60% to 80%, preferably, in concentration of 70%, like step (2).

If the fatty acid-urea inclusion compounds are not applied as the secondary nucleuses, it has an enormous effect on the results of the process such as purity, recover rate and the like. That is, in such case, urea crystals are precipitated at temperature lower than the case of using the fatty acid-urea inclusion compounds since it requires a considerable induction time to be crystallized from the micro molecular level. Accordingly, the precipitation of urea crystals is unavoidable if not applying the fatty acid-urea inclusion compounds as the secondary nucleation in such system that the temperature difference between the crystallization of urea inclusion compounds and the precipitation of urea crystals is within about 5°C. Consequently, the second nucleation mechanism using fatty acid-urea inclusion compounds in accordance with the present invention can maximize the resolving power, the recover rate and the utilization of urea.

Besides, since the present invention can isolate and purify unsaturated fatty acids without the precipitation of urea inclusion compounds in a high purity even at a high cooling rate (i.e., 0.5 to 1°C/min).

According to the present invention, it is possible to isolate and purify unsaturated fatty acids in a high purity of at least 99and via the secondary nucleation mechanism using fatty acid-urea inclusion compounds, the unsaturated fatty acids having valuable effects to be applied for the food, cosmetic and medicinal purpose.

### BRIEF DESCRIPTION OF THE DRAWINGS

Fig. 1 is a flowchart showing a method for preparing unsaturated fatty acids in a high purity from fat and oil fatty acids in accordance with the present invention;
Fig. 2 shows the analysis result of conjugated linoleic acids obtained according to a preferred embodiment of the invention by a gas chromatography analyzer;
Fig. 3 shows scanning electron microscope (SEM) images of a fatty acid-urea inclusion compound according to the invention and urea measured by SEM; and
Fig. 4 shows graphs of X-ray diffraction (XRD) data of a fatty acid-urea inclusion compound according to the invention and urea measured by XRD.

### DETAILED DESCRIPTION OF THE INVENTION

Hereinafter, preferred embodiments of the present invention will be described in detail with reference to the attached drawings. The present invention is not restricted to the following embodiments, and many variations are possible within the spirit and scope of the present invention. The embodiments of the present invention are provided in order to more completely explain the present invention to anyone skilled in the art.

### Embodiment 1: Preparation of Conjugated Linoleic Acid

### 1. Conversion of Conjugated Linoleic Acid

After adding 500ml of propylene glycol to a synthetic reactor, where a temperature of 175°C was maintained, if the temperature came down to 130°C, 250g of NaOH was added to the synthetic reactor and completely dissolved. Next, the resulting solution was stirred until the temperature came up to 175°C. 1kg of Safflower oil (linoleic acid of 75%) was added to the solution to carry out a conjugated isomerization reaction for one and a half hours. Then, after cooling the solution at a temperature of 50°C, the conjugated isomerization reaction was terminated. When the temperature came down to 50°C, phosphoric acid was added slowly to the reactant to adjust the pH value to 1. After stationing under this condition, the lower part of the reactant was removed and the conjugated linoleic acid layer in the upper part was washed by distilled water. This process was executed two to three times to remove impurities dissolved in water completely. 952g of the conjugated linoleic acids (95.2%) obtained in this step contain unreacted linoleic acids of 0.5% or less and trans-9,trans-11-conjugated linoleic acids of 0.8% or less.

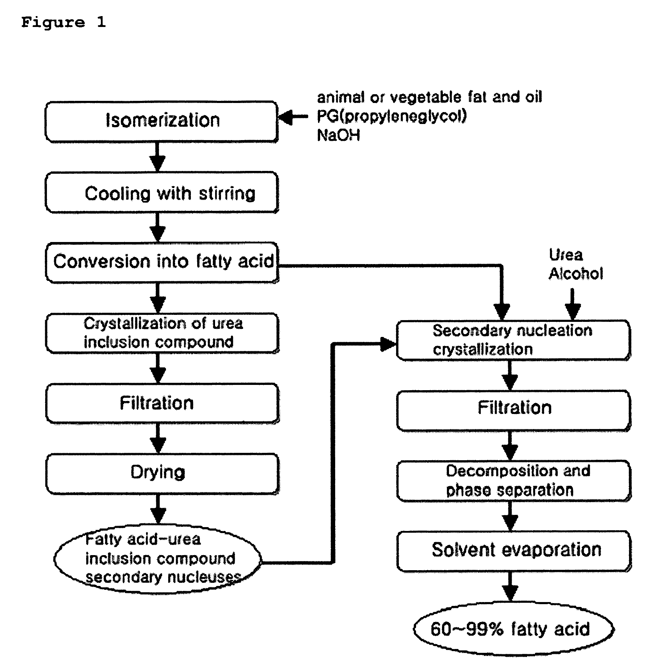

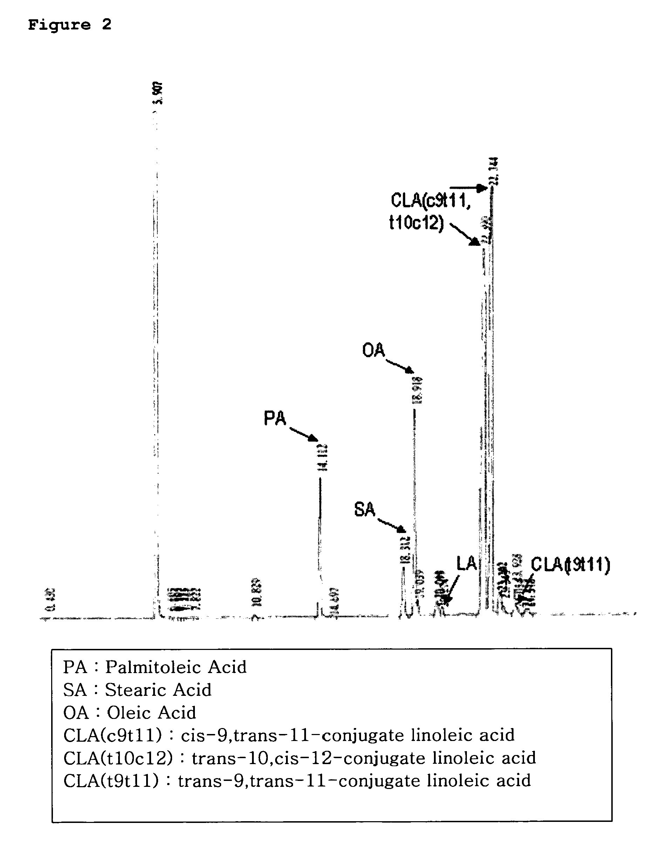

The method for preparing unsaturated fatty acids in a high purity from fat and oil fatty acids in accordance with the present invention is described in a flowchart of Fig. 1. Fig. 2 shows the analysis result of the conjugated linoleic acids obtained in step 1 using a gas chromatography analyzer.

As shown in Fig. 2, the conjugated linoleic acid converted contains palmitoleic acid of 6.59%, stearic acid of 2.86%, oleic acid of 14.20%, cis-9,trans-11 conjugated linoleic acid of 37.36%, trans-10,cis-12 conjugated linoleic acid of 38.91% and trans-9,trans-11 conjugated linoleic acid of 0.08%. Accordingly, it can be understood that the method for preparing unsaturated fatty acids of the present invention can minimize the formation of trans-9,trans-11 conjugated linoleic acids, which are carcinogenic substances.

### 2. Preparation of Conjugated Linoleic Acid-Urea

### Inclusion Compound Crystals

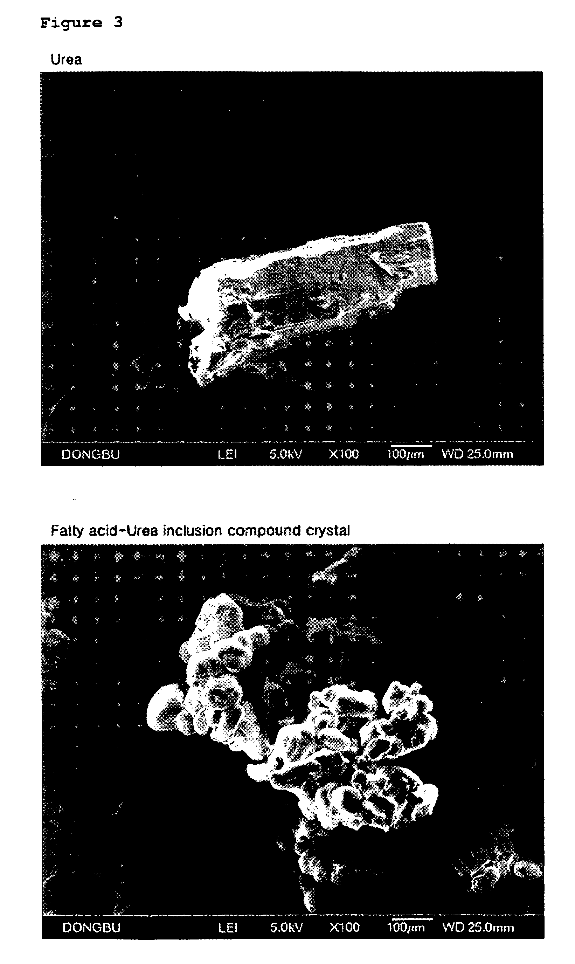

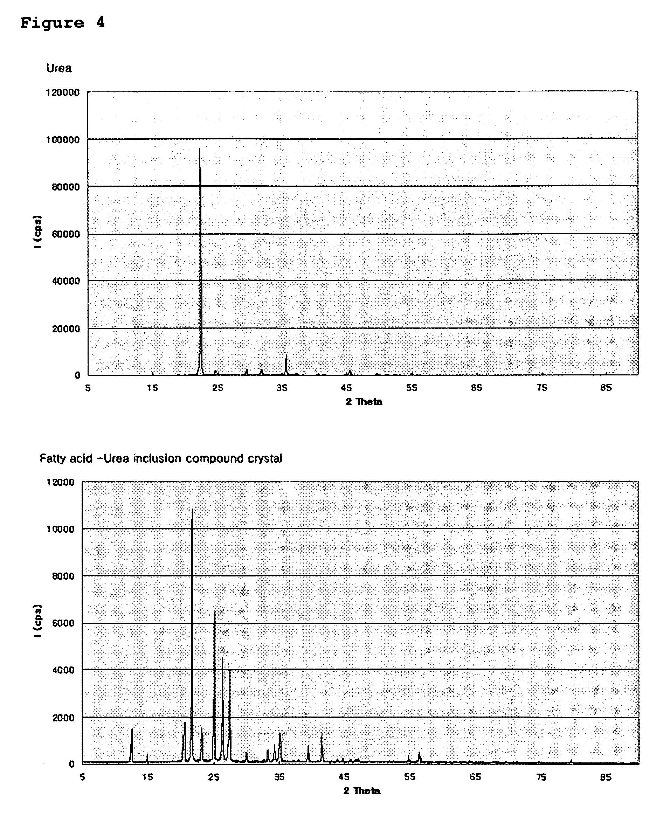

3kg of urea was added to 9L of ethanol solution of 70% and completely dissolved at temperature of 25°C to 30°C. Keeping this temperature, 952g of conjugated linoleic acids obtained in step 1 were added to the resulting solution to form conjugated linoleic acid-urea inclusion compound crystals. Next, the solution was filtered under reduced pressure and the portion of crystals was dried at a temperature of 50°C. The crystals were used as nucleuses in the following secondary nucleation mechanism. Crystals obtained in this step were measured by SEM images and XRD data in Figs. 3 and 4.

As shown in Fig. 3, it was found that urea itself has a rectangular shape (tetragonal system) and fatty acid-urea inclusion compound crystal has a hexagonal shape (system).

Besides, according to Joint Committee for Powder Diffraction Studies (JCPDS) card, it is known that urea itself has a rectangular shape, while fatty acid-urea inclusion compound crystal has a hexagonal shape. As shown in Fig. 4, since it was found that fatty acid-urea inclusion compound crystal obtained according to the invention has a hexagonal shape, it can be understood that crystals were formed in this step.

### 3. Preparation of Conjugated Linoleic Acid

4kg of urea and 1kg of conjugated linoleic acid were added to 12L of ethanol solution of 70% and completely dissolved at a temperature of 70°C. Then, the resulting solution was cooled at a cooling rate of 0.7°C/min. When the temperature came down to 50°C, 200g of conjugated linoleic acid-urea inclusion compound crystals formed in step 2 were added to the cooled solution. Here, it is most desired that the crystals are not completely melted but keep their crystal shapes. Under the condition that the crystals were present, the resulting solution was cooled at a cooling rate of 0.7°C/min to 10°C. Then, the portion of crystals was filtered under reduced pressure and collected. When 5kg of water, 2ml of hexane and a small quantity of hydrochloric acid were added to the resulting solution and stirred, conjugated linoleic acids containing hexane were formed in the upper part thereof, thus obtaining 930g of concentrated conjugated linoleic acids (93.0%) by removing the hexane from the upper part.

### Embodiment 2 Preparation of Gamma Linolenic Acid

Gamma linolenic acid-urea inclusion compound crystals were obtained using borage oil fatty acids (gamma linolenic acid of 20%) instead of the conjugated linoleic acids in the same manner in steps 1 and 2 of Embodiment 1.

4kg of urea and 1kg of borage oil fatty acid (gamma linolenic acid of 20%) were added to 12L of ethanol solution of 70% and completely dissolved at a temperature of 70°C. Then, the resulting solution was cooled at a cooling rate of 0.7°C/min. When the temperature came down to 50°C, 250g of gamma linolenic acid-urea inclusion compound crystals formed in the above step were added to the cooled solution. Under the condition that the crystals were present, not melted, the resulting solution was cooled at a cooling rate of 0.5°C/min till the temperature came down to 15°C. Next, the portion of crystals was filterd under reduced pressure and and discarded and, then, ethanol in the filtrate was evaporated using a rotary vacuum evaporator. Subsequently, when 1kg of water, 0.5ml of hexane and a small quantity of hydrochloric acid were added to the resulting solution and stirred, gamma linolenic acids containing hexane were formed in the upper part thereof, thus obtaining 170g of concentrated conjugated linoleic acids (99.3%) when removing the hexane in the upper part.

### Embodiment 3 Preparation of Docahexaenoic Acid (DHA)

DHA-urea inclusion compound crystals were prepared using microorganism fermented milk (DHA 40%) instead of the conjugated linoleic acids in the same manner in steps 1 and 2 of Embodiment 1.

3kg of urea and 1kg of microorganism fermented milk (DHA 40%) were added to 91 of ethanol solution of 70% and completely dissolved at a temperature of 70°C. Then, the resulting solution was cooled at a cooling rate of 0.6°C /min. When the temperature came down to 50°C, 200g of DHA-urea inclusion compound crystals formed in the above step were added to the cooled solution. Under the condition that the crystals were present, not melted, the resulting solution was cooled at a cooling rate of 0.5°C/min till the temperature came down to 10°C. Next, the portion of crystals was filtered under reduced pressure and discarded and, then, ethanol in the filtrate was evaporated using the rotary vacuum evaporator. Subsequently, when 1kg of water, 0.5ml of hexane and a small quantity of hydrochloric acid were added to the resulting solution and stirred, DHAs containing hexane were obtained in the upper part thereof, thus obtaining 150g of concentrated DHAs (99.1%) when removing the hexane in the upper part.

### Experimental Example: Fatty Acid Composition Analysis

An experiment, which will be described below, was carried out to identify the purities of the fatty acids in accordance with the present invention.

Compositions of gamma linolenic acid and DHA prepared according to Embodiments 2 and 3, respectively, were analyzed using a gas chromatography analyzer (Shimadzu cp 9001, Chrompack).

As comparative examples, gamma linolenic acid (GLA) and DHA prepared by the method disclosed in Korean Patent Laid-Open No. 10-2002-42432 were taken.

The result is shown in Table 1

**[Table 1]**

| | | Present Invention | | | | Comparative Examples | | | |
|---|---|---|---|---|---|---|---|---|---|
| Method Applied | | Embodiment 2 (GLA) | | Embodiment 3 (DHA) | | GLA | | DHA | |
| | Urea Inclusion Compound | Oleic Acid: | 0.0% | DPA: | 0.9% | Oleic Acid: | 0.6% | Oleic Acid: | 6.3% |
| | | Linoleic Acid: | 0.7% | DHA: | 99.1% | Linoleic Acid: | 12.3% | Linoleic Acid: | 8.1% |
| | | GLA: | 99.3% | | | GLA: | 87.1% | GLA: | 85.6% |
| | Cooling Crystallization | Not Applied | | Not Applied | | Linoleic Acid: | 1.8% | GLA: | 1.5% |
| | | | | | | | | DPA: | 2.3% |
| | | | | | | GLA: | 98.2% | DHA: | 96.2% |
| | Chromato- graphy | Not Applied | | Not Applied | | Linoleic Acid: | 0.9% | DpA: | 0.9% |
| | | | | | | GLA: | 99.1% | DHA: | 99.1% |

As shown in Table 1, when the urea inclusion compound method is applied, gamma linolenic acids and DHAs according to the invention were obtained 99.3% and 99.1%, whereas, those according to the comparative examples were 87.1% and 85.6%, respectively. As described above in detail, it is possible to achieve fatty acids in a purity of at least 99% using only the urea inclusion compound method. However, in the comparative examples, fatty acids in a purity of at least 99% may be obtained only when using the cooling crystallization and chromatography at the same time.

In the conventional method, since the urea inclusion compounds were cooled slowly for more than one day, the rancidity proceeded rapidly and, accordingly, the oxidative stability of fatty acids decreased. To improve the rancidity in the comparative examples, fatty acids were added to a mixture of alcohol and urea by parcels for five to six hours. As a result, urea and urea inclusion compound are simultaneously precipitated in the form of crystal, thus failing in obtaining fatty acids in a high purity. However, since urea and urea inclusion compound coexist in the cluster, the method according to the invention controls the behavior of the urea molecular group in order not to be crystallized but to be returned to atomic states using Ostwald ripening related to the growth of larger crystals from those of smaller size which have a higher solubility than the larger ones, such that the urea molecules cling to fatty acids to form fatty acid-urea inclusion compounds. Here, water and hexane in the weight ratio of 1:1 and a small amount of hydrochloric acid are added to the fatty acid-urea inclusion compounds to be stirred, thus obtaining fatty acids in a high purity.

Consequently, according to the invention, it is possible to preparing fatty acids in a high purity using only the urea inclusion compound method, not adopting the cooling crystallization and the chromatography.

The method for preparing unsaturated fatty acids of the present invention controls the behavior of the urea molecular group via a secondary nucleation mechanism using fatty acid-urea inclusion compounds. Accordingly, since the method of the invention converts fatty acids into urea inclusion compounds completely without precipitation of urea crystals at a high cooling rate, it is possible to prepare unsaturated fatty acids in a mass production by shortening the staying time at high temperature. Besides, the method of the invention can enhance the oxidative stability and the selectivity of fatty acids sharply.

## Claims

1. A method for preparing unsaturated fatty acids comprising the steps of:
(1) putting an NaOH into a C₁~C₅ alcohol to be completely dissolved, adding a fat and oil fatty acid to the resulting solution and cooling the resulting reactant to be converted into a fatty acid;
(2) adding a urea to an ethanol solution to be completely dissolved and adding the fatty acid obtained in step (1) to the resulting solution to form a fatty acid-urea inclusion compound; and
(3) stirring the ethanol solution, the urea and the fatty acid to be completely dissolved, cooling the resulting solution and, at the same time, adding the fatty acid-urea inclusion compound obtained in step (2) to the resulting reactant to obtain the fatty acid.

2. The method for preparing unsaturated fatty acids as recited in claim 1, wherein the alcohol in step (1) is a propylene glycol

3. The method for preparing unsaturated fatty acids as recited in claim 1, wherein the ethanol solution has a concentration of 60% to 80%.

4. The method for preparing unsaturated fatty acids as recited in claim 3, wherein the ethanol solution has a concentration of 70%.

5. The method for preparing unsaturated fatty acids as recited in claim 1, wherein the fat and oil fatty acid in step (1) is an animal or a vegetable fat and oil fatty acid comprising at least one selected from the group consisting of a safflower oil, a sunflower oil, an olive oil, and a sardine oil.

6. The method for preparing unsaturated fatty acids as recited in claim 1, wherein the fatty acid comprises at least one selected form the group consisting of a myristoleic acid, a palmitoleic acid, a γ-linolenic acid, an α-linolenic acid, an oleic acid, a linoleic acid, a conjugated linoleic acid, a docosahexaenoic acid(DHA) and an eicosapentaenoic acid(EPA).

7. The method for preparing unsaturated fatty acids as recited in claim 1, wherein in step (1) 0.25 to 0.5 weight fractions of NaOH are added to 1.0 weight fraction of propylene glycol to be completely dissolved and 1.0 to 2.0 weight fractions of fat and oil fatty acid are added to the solution to react and, the resulting reactant is cooled at a temperature of 50°C to 60°C.

8. The method for preparing unsaturated fatty acids as recited in claim 1, wherein after carrying out step (1) the fatty acid includes a unreacted fatty acid of 0.2% to 0.5% and a trans-9,trans-11 fatty acid of 1.0% or less.

9. The method for preparing unsaturated fatty acids as recited in claim 1, wherein in step (2) 1.0 weight fraction of urea is mixed with 3.0 to 5.0 weight fractions of ethanol solution to be completely dissolved and 0.2 to 0.4 weight fractions of the fatty acid obtained in step (1) is added to the solution to form a fatty acid-urea inclusion compound.

10. The method for preparing unsaturated fatty acids as recited in claim 1, wherein in step (3) 9.0 to 12.0 weight fractions of ethanol solution, 3.0 to 4.0 weigh parts of urea and 1.0 weight fraction of fatty acid are stirred to be completely dissolved and the solution is cooled at a cooling rate of 0.5 to 1.0°C/min, to which 0.01 to 0.05 weight fractions of the fatty acid-urea inclusion compound obtained in step (2) are added to form a fatty acid.
